(19) Europäisches Patentamt
European Patent Office
Office européen des brevets

(11) Veröffentlichungsnummer: **0 391 186**
**A1**

(12) # EUROPÄISCHE PATENTANMELDUNG

(21) Anmeldenummer: **90105653.1**

(51) Int. Cl.5: **C07D 277/68, A01N 43/78**

(22) Anmeldetag: **24.03.90**

(30) Priorität: **07.04.89 DE 3911227**

(43) Veröffentlichungstag der Anmeldung:
**10.10.90 Patentblatt 90/41**

(84) Benannte Vertragsstaaten:
**BE CH DE FR GB IT LI NL**

(71) Anmelder: **BAYER AG**

**D-5090 Leverkusen 1 Bayerwerk(DE)**

(72) Erfinder: **Wagner, Klaus, Dr.**
**Jakob-Böhme-Strasse 2**
**D-5000 Köln 80(DE)**
Erfinder: **Hänssler, Gerd, Dr.**
**Am Arenzberg 58a**
**D-5090 Leverkusen 3(DE)**
Erfinder: **Brandes, Wilhelm, Dr.**
**Eichendorffstrasse 3**
**D-5653 Leichlingen 1(DE)**

(54) **Nitrosubstituierte Benzthiazolone, Verfahren zu ihrer Herstellung und ihre Verwendung in Schädlingsbekämpfungsmitteln und neue Zwischenprodukte.**

(57) Nitrosubstituierte Benzthiazolone der Formel (I)

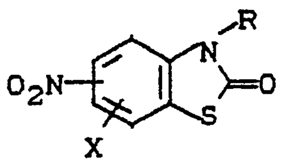

(I)

in welcher
X und R die in der Beschreibung gegebene Bedeutung haben, ihre Verwendung zur Bekämpfung von Schädlingen und neue Zwischenprodukte.

Die neuen Verbindungen der Formel (I) können nach Analogieverfahren hergestellt werden, z.B. aus geeigneten nitrosubstituierten Benzthiazolonen mit geeigneten Halogenverbindungen oder aus geeigneten nitrosubstituierten Benzthiazol-3-oxiden mit Phosphorylchlorid. Einige der Ausgangsverbindungen sind ebenfalls neu und auch nach Analogieverfahren herstellbar.

EP 0 391 186 A1

## Nitrosubstituierte Benzthiazolone, Verfahren zu ihrer Herstellung und ihre Verwendung in Schädlingsbekämpfungsmitteln und neue Zwischenprodukte

Die vorliegende Erfindung betrifft neue nitrosubstituierte Benzthiazolone, Verfahren zu ihrer Herstellung, ihre Verwendung in Schädlingsbekämpfungsmitteln und neue Zwischenprodukte.

Es ist bereits bekannt, daß man bestimmte Pflanzenkrankheiten durch cyclische Schwefelverbindungen bekämpfen kann. So kann z.B. 6-Methyl-2,3-chinoxalindithiol-cyclocarbonat (Chinomethionat/Morestan) gegen Mehltau im Obstbau verwendet werden (vgl. DE-AS 1 100 372). Die Wirkung dieser bekannten Verbindung ist jedoch, insbesondere bei niedrigen Wirkstoffkonzentrationen, nicht immer zufriedenstellend.

Weiter sind durch Nitro- und/oder Trifluormethyl-substituierte Benzthiazolone bekannt geworden (vgl. Chem. Ber. 107 (1974), 305-315; DE-OS 2 101 150), die ebenfalls eine biologische Wirkung haben.

Es wurden nun neue nitrosubstituierte Benzthiazolone der allgemeinen Formel (I)

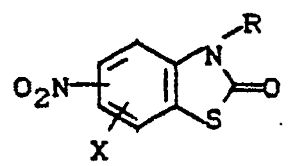

(I)

in welcher

R für Alkyl steht, welches einfach oder zweifach, gleich oder verschieden durch Cyano, Carboxy, Alkylcarbonyl, Alkoxycarbonyl und Alkylaminocarbonyl oder durch jeweils gegebenenfalls ein- bis fünffach, gleich oder verschieden durch Halogen, Alkyl, Halogenalkyl und Alkoxy substituiertes Phenyl oder Phenylcarbonyl subsitituiert ist, oder für den Rest -CONHR$^1$ steht, worin R$^1$ für Alkyl steht, welches im Alkylteil durch Cyano, Carboxy oder Alkoxycarbonyl einfach oder zweifach, gleich oder verschieden substituiert ist, und

X für Wasserstoff, Halogen, Alkyl oder Halogenalkyl steht,

gefunden.

Weiter wurde gefunden, daß man die neuen nitrosubstituierten Benzthiazolone der allgemeinen Formel (I)

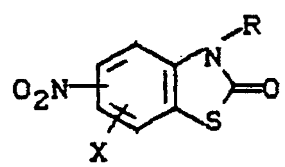

(I)

in welcher

R für Alkyl steht, welches einfach oder zweifach, gleich oder verschieden durch Cyano, Carboxy, Alkylcarbonyl, Alkoxycarbonyl und Alkylaminocarbonyl oder durch jeweils gegebenenfalls ein- bis fünffach, gleich oder verschieden durch Halogen, Alkyl, Halogenalkyl und Alkoxy substituiertes Phenyl oder Phenylcarbonyl substituiert ist, oder für den Rest -CONHR$^1$ steht, wobei R$^1$ für Alkyl steht, welches im Alkylteil durch Cyano, Carboxy oder Alkoxycarbonyl substituiert ist, und

X für Wasserstoff, Halogen, Alkyl oder Halogenalkyl steht,

erhält man, wenn man

(a) nitrosubstituierte Benzthiazolone der allgemeinen Formel (II)

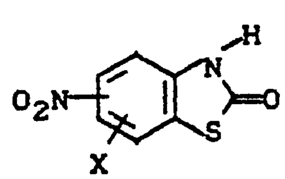

(II)

in welcher

X die oben angegebene Bedeutung hat,

- oder Alkalimetallsalze von Verbindungen der allgemeinen Formel (II) -
mit Halogenverbindungen der allgemeinen Formel (III)

$X^1$-R (III)

in welcher
R die oben angegebene Bedeutung hat und
$X^1$ für Halogen steht,
gegebenenfalls in Gegenwart eines Säureakzeptors und gegebenenfalls in Gegenwart eines Verdünnungsmittels umsetzt, oder wenn man

(b) nitrosubstituierte Benzthiazol-3-oxide der allgemeinen Formel (IV)

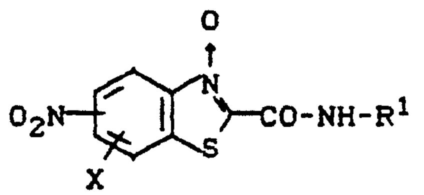

(IV)

in welcher
X und $R^1$ die oben angegebenen Bedeutungen haben,
mit Phosphorylchlorid ($POCl_3$) gegebenenfalls in Gegenwart eines Verdünnungsmittels umsetzt.

Die neuen nitrosubstituierten Benzthiazolone der allgemeinen Formel (I) weisen interessante biologische Eigenschaften auf; sie zeichnen sich insbesondere durch eine starke fungizide Wirkung aus.

Überraschenderweise zeigen die erfindungsgemäßen Verbindungen der Formel (I) z.B. eine erheblich stärkere fungizide Wirkung als das bekannte Fungizid 6-Methyl-2,3-chinoxalindithiol-cyclocarbonat.

Die erfindungsgemäßen nitrosubstituierten Benzthiazolone sind durch die Formel (I) allgemein definiert.
Bevorzugt sind Verbindungen der Formel (I),
in welcher
R für geradkettiges oder verzweigtes Alkyl mit 1 bis 6 Kohlenstoffatomen steht, welches einfach oder zweifach, gleich oder verschieden durch Cyano, Carboxy, $C_1$-$C_4$-Alkyl-carbonyl, $C_1$-$C_4$-Alkoxy-carbonyl und $C_1$-$C_4$-Alkylamino-carbonyl oder durch jeweils gegebenenfalls ein- bis dreifach, gleich oder verschieden durch Halogen, $C_1$-$C_4$-Alkyl, $C_1$-$C_4$-Halogenalkyl mit 1 bis 9 gleichen oder verschiedenen Halogenatomen und $C_1$-$C_4$-Alkoxy substituiertes Phenyl oder Phenylcarbonyl substituiert ist, oder für den Rest -CONH$R^1$ steht, wobei $R^1$ für Alkyl mit 1 bis 6 Kohlenstoffatomen im geradkettigen oder verzweigten Alkyl steht, welches ein- oder zweifach, gleich oder verschieden durch Cyano, Carboxy oder $C_1$-$C_4$-Alkoxy-carbonyl substituiert ist, und
X für Wasserstoff, Halogen, $C_1$-$C_4$-Alkyl oder $C_1$-$C_4$-Halogenalkyl mit 1 bis 9 gleichen oder verschiedenen Halogenatomen steht.

Besonders bevorzugt sind Verbindungen der Formel (I), in welcher
R für Methyl oder Ethyl steht, welches einfach oder zweifach, gleich oder verschieden durch Cyano, Carboxy, $C_1$-$C_4$-Alkyl-carbonyl, $C_1$-$C_4$-Alkoxy-carbonyl, oder durch jeweils ein- bis dreifach, gleich oder verschieden durch Fluor, Chlor, Brom, Methyl, Ethyl, Trifluormethyl, Methoxy und Ethoxy substituiertes Phenyl oder Phenylcarbonyl substituiert ist, oder für den Rest -CONH$R^1$ steht, worin $R^1$ für Alkyl mit 1 oder 2 Kohlenstoffatomen steht, welches ein-oder zweifach durch Cyano, Carboxy oder $C_1$-$C_4$-Alkoxy-carbonyl substituiert ist, und
X für Wasserstoff, Chlor, Methyl oder Trifluormethyl steht.

Halogen steht für Fluor, Chlor, Brom oder Jod, bevorzugt für Fluor, Chlor oder Brom, besonders bevorzugt für Fluor oder Chlor allein oder in Zusammensetzung wie Halogenalkyl, wenn nicht anders definiert.

Alle Reste, die substituiert sein können, sind ein- bis mehrfach, gleich oder verschieden, vor allem ein- bis fünffach, bevorzugt ein- bis dreifach und besonders bevorzugt ein- oder zweifach substituiert, wenn nicht anders angegeben.

Verwendet man beispielsweise 6-Nitro-benzthiazolon und 4-Fluor-benzylchlorid als Ausgangsstoffe, so kann der Reaktionsablauf beim erfindungsgemäßen Verfahren (a) durch das folgende Formelschema wiedergegeben werden:

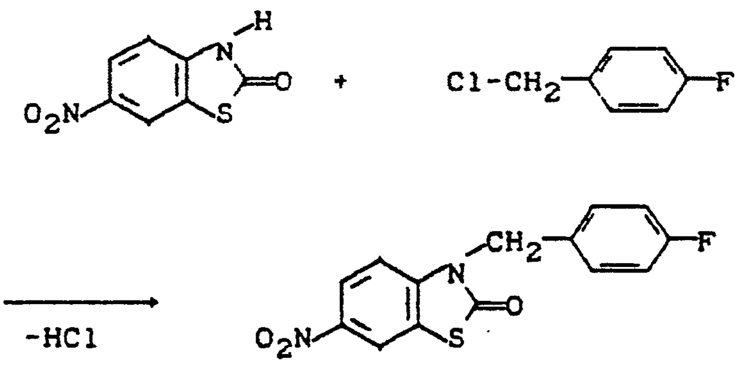


Verwendet man beispielsweise 5-Methyl-7-nitro-2-cyanomethylaminocarbonyl-benzthiazol-3-oxid und Phosphorylchlorid als Ausgangsstoffe, so kann der Reaktionsablauf beim erfindungsgemäßen Verfahren (b) durch das folgende Formelschema wiedergegeben werden:

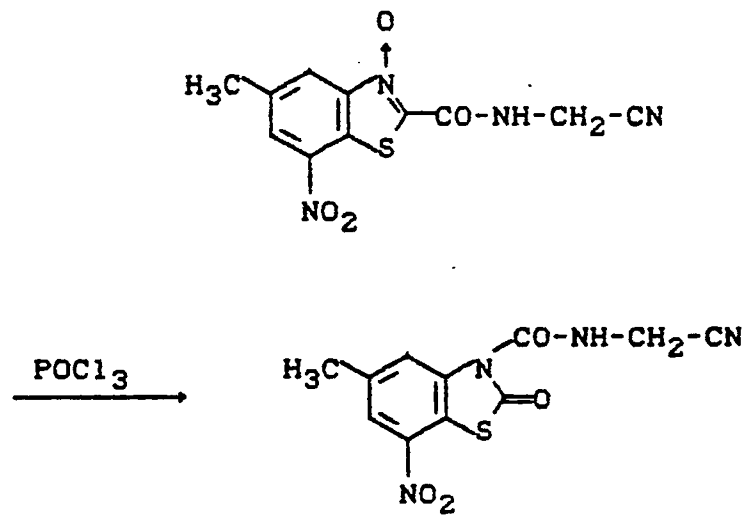


Die beim erfindungsgemäßen Verfahren (a) zur Herstellung von Verbindungen der Formel (I) als Ausgangsstoffe zu verwendenden nitrosubstituierten Benzthiazolone sind durch die Formel (II) allgemein definiert.

In der Formel (II) hat X vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für X angegeben wurde.

Als Beispiele für die Ausgangsstoffe der Formel (II) seien genannt:

4-Nitro-, 5-Nitro-, 6-Nitro- und 7-Nitro-benzthiazolon, 4-Chlor-5-nitro-, 4-Chlor-6-nitro-, 4-Chlor-7-nitro-, 5-Chlor-4-nitro-, 5-Chlor-6-nitro-, 5-Chlor-7-nitro-, 6-Chlor-4-nitro-, 6-Chlor-5-nitro-, 6-Chlor-7-nitro-, 7-Chlor-4-nitro-, 7-Chlor-5-nitro-und 7-Chlor-6-nitro-benzthiazolon, 4-Methyl-5-nitro-, 4-Methyl-6-nitro-, 4-Methyl-7-nitro-, 5-Methyl-4-nitro-, 5-Methyl-6-nitro-, 5-Methyl-7-nitro-, 6-Methyl-4-nitro-, 6-Methyl-5-nitro-, 6-Methyl-7-nitro-, 7-Methyl-4-nitro-, 7-Methyl-5-nitro- und 7-Methyl-6-nitrobenzthiazolon sowie 4-Trifluormethyl-5-nitro-, 4-Trifluormethyl-6-nitro-, 4-Trifluormethyl-7-nitro-, 5-Trifluormethyl-4-nitro-, 5-Trifluormethyl-6-nitro-, 5-Trifluormethyl-7-nitro-, 6-Trifluormethyl-4-nitro-, 6-Trifluormethyl-5-nitro-, 6-Trifluormethyl-7-nitro-, 7-Trifluormethyl-4-nitro-, 7-Trifluormethyl-5-nitro- und 7-Trifluormethyl-6-nitro-benzthiazolon.

Die Ausgangsstoffe der Formel (II) sind bekannt und/oder können nach an sich bekannten Verfahren hergestellt werden (vgl. Chem. Ber. 107 (1974), 305-316; Egypt. J.Chem. 16 (1973), 355-359; DE-OS 2 101 150; JP-A 60-105 671; EP-A 218 972).

Die beim erfindungsgemäßen Verfahren (a) weiter als Ausgangsstoffe zu verwendenden Halogenverbindungen sind durch die Formel (III) allgemein definiert.

In der Formel (III) hat R vorzugsweise bzw. insbesondere diejenige Bedeutung, die bereits oben im Zusammenhang mit der Beschreibung der erfindungsgemäßen Verbindungen der Formel (I) vorzugsweise

bzw. als insbesondere bevorzugt für R angegeben wurde und X steht für Chlor oder Brom.

Als Beispiele für die Ausgangsstoffe der Formel (III) seien genannt:

Chloraceton, Chlormethyl- und Brommethyl-tert.-butylketon, α-Chlor- und α-Brom-acetophenon, Chlor- und Bromacetonitril sowie -α- und -β-propionitril, Chlor- und Brom-essigsäure sowie -α- und -β-propionsäure sowie deren Methylester, Ethylester, Propylester, Isopropylester, Butylester und Isobutylester, Chlor- und Brommalodinitril, Chlor- und Brom-cyanessigsäure sowie deren Methylester, Ethylester, Propylester, Isopropylester, Butylester und Isobutylester, Chlor- und Brom-malonsäure sowie deren Dimethylester, Diethylester, Dipropylester, Diisopropylester, Dibutylester und Diisobutylester, 2-Chlor-, 3-Chlor-, 4-Chlor-, 2,4-Dichlor-, 2,5-Dichlor-, 2,6-Dichlor-, 3,4-Dichlor- und 3,5-Dichlor-benzylchlorid, 2-Fluor-, 3-Fluor-, 4-Fluor-, 2,4-Difluor- und 3,4-Difluor-benzylchlorid, 2-Methyl-, 3-Methyl-, 4-Methyl- und 3,4-Dimethyl-benzylchlorid, 2-Trifluormethyl-, 3-Trifluormethyl- und 4-Trifluormethyl-benzylchlorid sowie 2-Methoxy-, 3-Methoxy-, 4-Methoxy- und 3,4-Dimethoxy-benzylchlorid.

Die Ausgangsstoffe der Formel (III) sind bekannte organische Synthesechemikalien.

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methylisobutyl-keton, Ester wie Essigsäuremethylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid ferner auch Alkohole wie Methanol, Ethanol, Propanol, Isopropanol, Butanol, Isobutanol, sec.-Butanol und tert.-Butanol.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalimetalle wie z.B. Natrium und Kalium, Alkalimetallhydroxide wie z.B. Natrium- und Kaliumhydroxid, Erdalkalihydroxide wie z.B. Calciumhydroxid, Alkalicarbonate und -alkoholate wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (a) in einem größeren Bereich variiert werden. Im allgemeinen arbeitet man bei Temperaturen zwischen 0 °C und 150 °C, vorzugsweise bei Temperaturen zwischen 10 °C und 120 °C.

Das erfindungsgemäße Verfahren (a) wird im allgemeinen unter Normaldruck durchgeführt. Es ist jedoch auch möglich, unter erhöhtem oder vemindertem Druck zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel durchgeführt und das Reaktionsgemisch wird mehrere Stunden bei der jeweils erforderlichen Temperatur gerührt. Die Aufarbeitung erfolgt bei dem erfindungsgemäßen Verfahren (a) jeweils nach üblichen Methoden.

Die beim erfindungsgemäßen Verfahren (b) zur Herstellung von Verbindungen der Formel (I) als Ausgangsstoffe zu verwendenden nitrosubstituierten Benzthiazol-3-oxide sind durch die Formel (IV) allgemein definiert.

In der Formel (IV) hat X vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für X angegeben wurde und

$R^1$ steht vorzugsweise für geradkettiges oder verzweigtes Alkyl mit 1 bis 6 Kohlenstoffatomen, welches durch Cyano, Carboxy oder $C_1$-$C_4$-Alkoxy-carbonyl substituiert ist,

insbesondere für Methyl oder Ethyl, welche durch Cyano, Carboxy oder $C_1$-$C_4$-Alkoxy-carbonyl substituiert sind.

Beispiele für die Ausgangsstoffe der Formel (IV) sind in der nachstehenden Tabelle 1 aufgeführt.

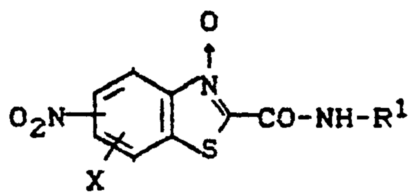

(IV)

<u>Tabelle 1:</u> Beispiele für die Ausgangsstoffe der Formel (IV)

| Position von $NO_2$ | (Position-)X | $R^1$ |
|---|---|---|
| 6 | - | $-CH_2CH_2CN$ |
| 6 | - | $-CH_2COOCH_3$ |
| 6 | - | $-CH(CH_3)COOC_2H_5$ |
| 6 | - | $-CH_2CH_2COOC_2H_5$ |
| 6 | - | $-CH_2COOH$ |
| 6 | - | $-CH_2COOC_2H_5$ |
| 6 | (7-)Cl | $-CH_2CH_2CN$ |
| 6 | (7-)Cl | $-CH_2COOH$ |
| 6 | (7-)Cl | $-CH_2COOCH_3$ |
| 6 | (7-)Cl | $-CH_2COOC_2H_5$ |
| 6 | (7-)Cl | $-CH_2CH_2COOC_2H_5$ |
| 6 | (7-)Cl | $-CH(CH_3)COOC_2H_5$ |
| 7 | (5-)Cl | $-CH_2CH_2CN$ |
| 7 | (5-)Cl | $-CH_2COOH$ |
| 7 | (5-)Cl | $-CH_2COOCH_3$ |
| 7 | (5-)Cl | $-CH_2COOC_2H_5$ |

Tabelle 1: (Fortsetzung)

| Position von $NO_2$ | (Position-)X | $R^1$ |
|---|---|---|
| 7 | (5-)Cl | $-CH_2CH_2COOC_2H_5$ |
| 7 | (5-)Cl | $-CH(CH_3)COOC_2H_5$ |
| 7 | (5-)$CF_3$ | $-CH_2CH_2CN$ |
| 7 | (5-)$CF_3$ | $-CH_2COOH$ |
| 7 | (5-)$CF_3$ | $-CH_2COOCH_3$ |
| 7 | (5-)$CF_3$ | $-CH_2COOC_2H_5$ |
| 7 | (5-)$CF_3$ | $-CH_2CH_2COOC_2H_5$ |
| 7 | (5-)$CF_3$ | $-CH(CH_3)COOC_2H_5$ |
| 7 | (5-)$CH_3$ | $-CH_2CH_2CN$ |
| 7 | (5-)$CH_3$ | $-CH_2COOH$ |
| 7 | (5-)$CH_3$ | $-CH_2COOCH_3$ |
| 7 | (5-)$CH_3$ | $-CH_2COOC_2H_5$ |
| 7 | (5-)$CH_3$ | $-CH_2CH_2COOC_2H_5$ |
| 7 | (5-)$CH_3$ | $-CH(CH_3)COOC_2H_5$ |
| 5 | (7-)$CF_3$ | $-CH_2CH_2CN$ |
| 5 | (7-)$CF_3$ | $-CH_2COOH$ |
| 5 | (7-)$CF_3$ | $-CH_2COOCH_3$ |
| 5 | (7-)$CF_3$ | $-CH_2COOC_2H_5$ |
| 5 | (7-)$CF_3$ | $-CH_2CH_2COOC_2H_5$ |
| 5 | (7-)$CF_3$ | $-CH(CH_3)COOC_2H_5$ |
| 5 | (7-)Cl | $-CH_2CH_2CN$ |
| 5 | (7-)Cl | $-CH_2COOH$ |
| 5 | (7-)Cl | $-CH_2COOCH_3$ |
| 5 | (7-)Cl | $-CH_2COOC_2H_5$ |
| 5 | (7-)Cl | $-CH_2CH_2COOC_2H_5$ |
| 5 | (7-)Cl | $-CH(CH_3)COOC_2H_5$ |

**Tabelle 1:** (Fortsetzung)

| Position von $NO_2$ | (Position-)X | $R^1$ |
|---|---|---|
| 7 | (5-)Cl | $-CH_2CH_2CN$ |
| 7 | (5-)Cl | $-CH_2COOH$ |
| 7 | (5-)Cl | $-CH_2COOCH_3$ |
| 7 | (5-)Cl | $-CH_2COOC_2H_5$ |
| 7 | (5-)Cl | $-CH_2CH_2COOC_2H_5$ |
| 7 | (5-)Cl | $-CH(CH_3)COOC_2H_5$ |

Die Ausgangsstoffe der Formel (IV) sind noch nicht aus der Literatur bekannt.

Man erhält die neuen nitrosubstituierten Benzthiazol-3-oxide der allgemeinen Formel (IV), wenn man entsprechende 2-Alkoxycarbonyl-benzthiazol-3-oxide der allgemeinen Formel (V)

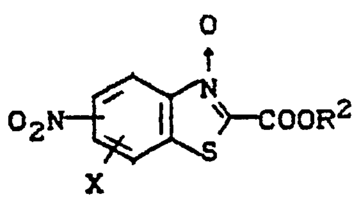

(V)

in welcher
X die oben angegebene Bedeutung hat und
$R^2$ für Alkyl, vorzugsweise mit 1 bis 4 Kohlenstoffatomen, steht
mit Aminen der allgemeinen Formel (VI)
$H_2N-R^1$ (VI)
in welcher
$R^1$ die oben angegebene Bedeutung hat,
gegebenenfalls in Gegenwart von Verdünnungsmitteln, wie z.B. Methanol oder Ethanol, bei Temperaturen zwischen 0° C und 100° C umsetzt.

Die als Zwischenprodukte benötigten 2-Alkoxycarbonylbenzthiazol-3-oxide der allgemeinen Formel (V), sind ebenfalls noch nicht aus der Literatur bekannt.

Man erhält die neuen 2-Alkoxycarbonyl-benzthiazol-3-oxide der allgemeinen Formel (V) wenn man entsprechende 2-Chlor-nitrobenzol-Derivate der allgemeinen Formel (VII)

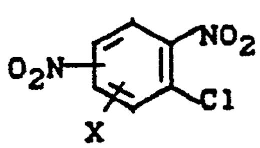

(VII)

in welcher
X die oben angegebene Bedeutung hat,
mit Mercaptoessigsäureestern der allgemeinen Formel (VIII)
$HS-CH_2-COOR^2$ (VIII)
in welcher
$R^2$ die oben angegebene Bedeutung hat,
in Gegenwart einer Base, wie z.B. Triethylamin, und in Gegenwart eines Verdünnungsmittels, wie z.B.

Dimethylsulfoxid, Benzol, Toluol, Tetrahydrofuran, Dioxan, Methanol, Ethanol, Propanol, Isopropanol und/oder Wasser, bei Temperaturen zwischen 0° C und 100° C, vorzugsweise zwischen 20° C und 80° C, umsetzt.

Die Ausgangsstoffe der Formeln (VI), (VII) und (VIII) sind bekannte organische Synthesechemikalien.

Das erfindungsgemäße Verfahren (b) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methylisobutylketon, Ester wie Essigsäuremethylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid.

Die Reaktionstemperaturen können bei dem erfindungsgemäßen Verfahren (b) in einem größeren Bereich variiert werden. Im allgemeinen arbeitet man bei Temperaturen zwischen 0° C und 150° C, vorzugsweise bei Temperaturen zwischen 20° C und 100° C.

Das erfindungsgemäße Verfahren (b) wird im allgemeinen bei Normaldruck durchgeführt.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel durchgeführt und das Reaktionsgemisch wird mehrere Stunden bei der jeweils er forderlichen Temperatur gerührt. Die Aufarbeitung erfolgt bei dem erfindungsgemäßen Verfahren jeweils nach üblichen Methoden.

Die erfindungsgemäßen Wirkstoffe weisen eine starke biologische Wirkung auf und können zur Bekämpfung von unerwünschten Schädlingen praktisch eingesetzt werden. Die Wirkstoffe sind z.B. für den Gebrauch als Pflanzenschutzmittel geeignet, vor allem zur Bekämpfung von Pilzen.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Verbindungen der Formel (I) zeigen insbesondere starke protektive Wirkung gegen Pyricularia-Arten, wie z.B. Pyricularia oryzae, welche im Reisanbau Schäden verursachen, sowie gegen Venturia-Arten, wie z.B. Venturia inaequalis, welche im Obstbau Schäden hervorrufen.

Auch gegen Plasmopara- und Fusarium-Arten wird eine gute Wirkung beobachtet.

Zum Teil zeigen die erfindungsgemäße Verbindungen der Formel (I) auch eine insektizide und akarizide Wirkung.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm

Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

## Herstellungsbeispiele

### Beispiel 1

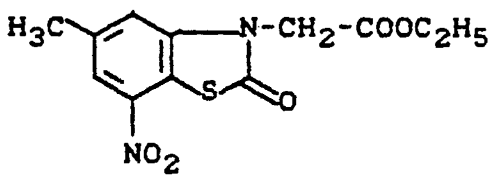

(Verfahren (a))

0,69 g (0,03 g-Atome) Natrium werden in 150 ml Ethanol gelöst. Zu dieser Lösung gibt man bei Raumtemperatur 6,3 g (0,03 Mol) 5-Methyl-7-nitro-benzthiazolon und versetzt anschließend mit 5 g (0,03 Mol) Bromessigsäureethylester. Der Reaktionsansatz wird 8 Stunden unter Rückfluß gekocht. Beim Abkühlen kristallisieren 7,8 g (87 % der Theorie) 5-Methyl-7-nitro-3-ethoxycarbonylmethyl-benzthiazolon in Form zitronengelber Nadeln vom Schmelzpunkt 154 °C aus.

### Beispiel 2

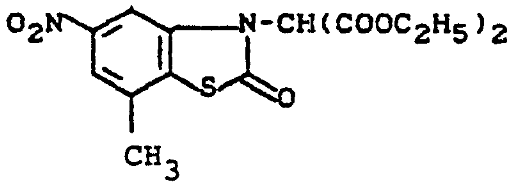

(Verfahren (a))

0,69 g (0,03 g-Atome) Natrium werden in 100 ml Ethanol gelöst. Man versetzt die Lösung mit 6,3 g (0,03 Mol) 5-Nitro-7-methyl-benzthiazolon und anschließend mit 7,17 g (0,03 Mol) Brommalonsäurediethylester. Der Reaktionsansatz wird 8 Stunden unter Rückfluß gekocht, auf Eis gegossen und das ausgeschiedene Produkt aus wenig Ethanol umkristallisiert. Man erhält 4 g (36 % der Theorie) 7-Methyl-5-nitro-3-(bis-ethoxycarbonyl-methyl)-benzthiazolon in Form farbloser Nadeln vom Schmelzpunkt 97 °C.

### Beispiel 3

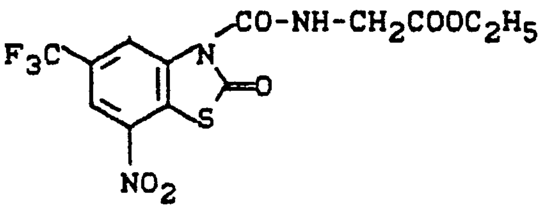

(Verfahren (b))

25 g (0,065 Mol) 2-Ethoxycarbonylmethylaminocarbonyl-5-trifluormethyl-7-nitro-benzthiazol-N-oxid werden in 150 ml Chloroform zum Sieden erhitzt. Bei dieser Temperatur läßt man 11 g (0,075 Mol) Phosphoroxychlorid eintropfen und rührt den Ansatz 4 Stunden bei 60 °C nach. Das beim Abkühlen der Reaktionsmischung sich abscheidende 5-Trifluormethyl-7-nitro-benzthiazolon wird abfiltriert und das Filtrat im Vakuum eingedampft. Der Rückstand wird aus Ethanol-Acetonitril umkristallisiert. Man gewinnt 10 g (39 % der Theorie) 5-Trifluormethyl-7-nitro-3-ethoxycarbonylmethylaminocarbonyl-benzthiazolon in Form farbloser Nadeln vom Schmelzpunkt 150 °C.

Analog zu den Beispielen 1 bis 3 und entsprechend der allgemeinen Beschreibung der erfindungsgemäßen Herstellungsverfahren können die in der nachstehenden Tabelle 2 aufgeführten Verbindungen der Formel (I) hergestellt werden.

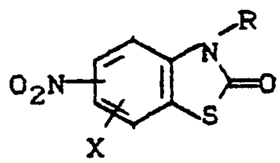

(I)

**T a b e l l e  2:** Beispiele für die Verbindungen der Formel (I)

| Beisp.-Nr. | Position von $NO_2$ | (Position-)X | R | Schmelzpunkt (°C) |
|---|---|---|---|---|
| 4 | 5 | (7-)$CF_3$ | $-CO-NH-CH_2COOC_2H_5$ | 119 |
| 5 | 5 | (7-)$CH_3$ | 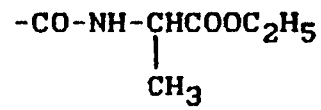 | 135 |
| 6 | 7 | (5-)$CF_3$ | $-CO-NH-CH_2CH_2CN$ | 135 |
| 7 | 7 | (5-)Cl | $-CH_2COOH$ | 252 |
| 8 | 7 | (5-)Cl | 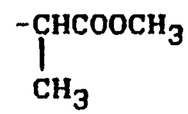 | 153 |
| 9 | 5 | (7-)$CF_3$ | 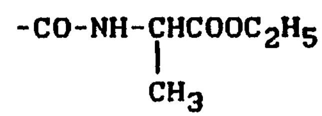 | 120 |
| 10 | 7 | (5-)Cl | $-CH(COOC_2H_5)_2$ | 104 |
| 11 | 7 | (5-)Cl | 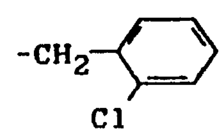 | 150 |

T a b e l l e 2: (Fortsetzung)

| Beisp.-Nr. | Position von $NO_2$ | (Position-)X | R | Schmelzpunkt (°C) |
|---|---|---|---|---|
| 12 | 7 | (5-)$CF_3$ | $-CH(COOC_2H_5)_2$ | 90 |
| 13 | 7 | (5-)$CF_3$ | $-CH_2-CO-CH_3$ | 202 |
| 14 | 7 | (5-)$CF_3$ | $-CH_2COOH$ | |
| 15 | 7 | (5-)$CF_3$ | $-CH_2$ 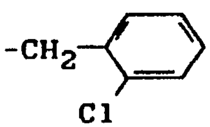 | 158 |
| 16 | 7 | (5-)$CF_3$ | $-CH_2CN$ | 158 |
| 17 | 7 | (5-)$CF_3$ | $-CH_2-CO-C(CH_3)_3$ | 158 |
| 18 | 5 | (7-)$CF_3$ | $-CH(COOC_2H_5)_2$ | 80 |
| 19 | 5 | (7-)$CF_3$ | $-CH_2-CO$ 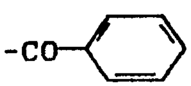 | 200 |
| 20 | 5 | (7-)$CF_3$ | $-CH_2-CO-C(CH_3)_3$ | 178 |

**T a b e l l e 2:** (Fortsetzung)

| Beisp.-Nr. | Position von $NO_2$ | (Position-)X | R | Schmelzpunkt (°C) |
|---|---|---|---|---|
| 21 | 7 | (5-)$CH_3$ | $-CH(COOC_2H_5)_2$ | 109 |
| 22 | 7 | (5-)$CH_3$ | $-CH_2$ 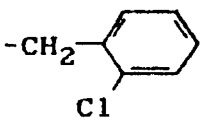 | 175 |
| 23 | 6 | (7-)Cl | $-CH(COOC_2H_5)_2$ | 125 |
| 24 | 7 | (5-)Cl | $-CH_2$ 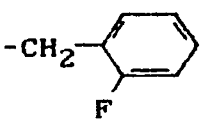 | 136 |
| 25 | 7 | (5-)Cl | $-CH_2$ 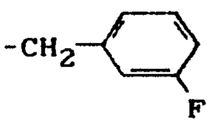 | 123 |
| 26 | 7 | (5-)Cl | $-CH_2$ 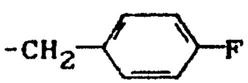 | 173 |
| 27 | 6 | - | $-CH(COOC_2H_5)_2$ | 102 |
| 28 | 5 | (7-)$CF_3$ | $-CH_2$ 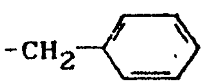 | 129 |

**Tabelle 2:** (Fortsetzung)

| Beisp.-Nr. | Position von $NO_2$ | (Position-)X | R | Schmelzpunkt (°C) |
|---|---|---|---|---|
| 29 | 5 | $(7-)CF_3$ | $-CH_2COOH$ | 205 |
| 30 | 7 | $(5-)CH_3$ | 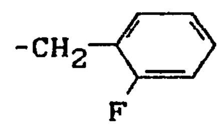 | 150 |
| 31 | 7 | $(5-)CH_3$ | 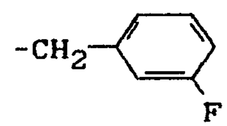 | 171 |
| 32 | 7 | $(5-)CH_3$ | 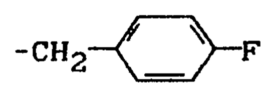 | 161 |
| 33 | 7 | $(5-)CH_3$ | 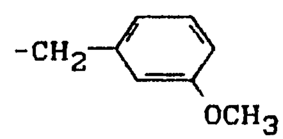 | 157 |

Tabelle 2: (Fortsetzung)

| Beisp.-Nr. | Position von $NO_2$ | (Position-)X | R | Schmelzpunkt (°C) |
|---|---|---|---|---|
| 34 | 7 | (5-)$CH_3$ | $-CH_2-$ 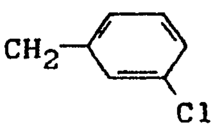 | 187 |
| 35 | 7 | (5-)$CH_3$ | $-CH_2-$ 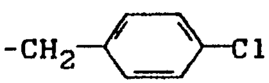 | 158 |
| 36 | 7 | (5-)$CH_3$ | $-CH_2-COOH$ | 250 |
| 37 | 7 | (5-)$CH_3$ | $-CH_2CN$ | 199 |

Anwendungsbeispiel

Im folgenden Anwendungsbeispiel wurde die nachstehend aufgeführte Verbindung als Vergleichssubstanz eingesetzt:

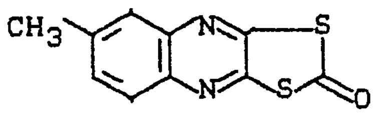

6-Methyl-2,3-chinoxalindithiol-cyclocarbonat (Chinomethionat) (vgl. DE-AS 1 100 372).

Beispiel A

Pyricularia-Test (Reis) /protektiv

Lösungsmittel: 12,5 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen (3), (6) und (10).

## Ansprüche

1. Nitrosubsituierte Benzthiazolone der allgemeinen Formel (I)

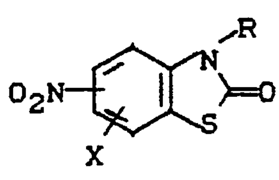

(I)

in welcher

R für Alkyl steht, welches einfach oder zweifach, gleich oder verschieden durch Cyano, Carboxy, Alkylcarbonyl, Alkoxycarbonyl und Alkylaminocarbonyl oder durch jeweils gegebenenfalls ein- bis fünffach, gleich oder verschieden durch Halogen, Alkyl, Halogenalkyl und Alkoxy substituiertes Phenyl oder Phenylcarbonyl substituiert ist, oder für den Rest -CONHR[1] steht, worin $R^1$ für Alkyl steht, welches ein- oder zweifach, gleich oder verschieden durch Cyano, Carboxy und Alkoxycarbonyl substituiert ist, und

X für Wasserstoff, Halogen, Alkyl oder Halogenalkyl steht.

2. Nitrosubstituierte Benzthiazolone der Formel (I) gemäß Anspruch 1,

in welcher
R für geradkettiges oder verzweigtes Alkyl mit 1 bis 6 Kohlenstoffatomen steht, welches einfach oder zweifach, gleich oder verschieden durch Cyano, Carboxy, $C_1$-$C_4$-Alkyl-carbonyl, $C_1$-$C_4$-Alkoxy-carbonyl und $C_1$-$C_4$-Alkylaminocarbonyl oder durch jeweils gegebenenfalls ein- oder dreifach, gleich oder verschieden durch Halogen, $C_1$-$C_4$-Alkyl, $C_1$-$C_4$-Halogenalkyl mit 1 bis 9 gleichen oder verschiedenen Halogenatomen und $C_1$-$C_4$-Alkoxy substituiertes Phenyl oder Phenylcarbonyl substituiert ist, oder für den Rest -CONHR$^1$ steht, worin R$^1$ für Alkyl mit 1 bis 6 Kohlenstoffatomen im geradkettigen oder verzweigten Alkyl steht, welches ein- oder zweifach, gleich oder verschieden durch Cyano, Carboxy oder $C_1$-$C_4$-Alkoxycarbonyl substituiert ist, und
X für Wasserstoff, Halogen, $C_1$-$C_4$-Alkyl oder $C_1$-$C_4$-Halogenalkyl mit 1 bis 9 gleichen oder verschiedenen Halogenatomen steht.

3. Nitrosubstituierte Benzthiazolone der Formel (I) gemäß Anspruch 1,
in welcher
R für Methyl oder Ethyl steht, welches einfach oder zweifach, gleich oder verschieden durch Cyano, Carboxy, $C_1$-$C_4$-Alkyl-carbonyl, $C_1$-$C_4$-Alkoxy-carbonyl, oder durch jeweils ein- bis dreifach, gleich oder verschieden durch Fluor, Chlor, Brom, Methyl, Ethyl, Trifluormethyl, Methoxy und Ethoxy substituiertes Phenyl oder Phenylcarbonyl substituiert ist, oder für den Rest -CONHR$^1$ steht, worin R$^1$ für Alkyl mit 1 oder 2 Kohlenstoffatomen steht, welches ein-oder zweifach durch Cyano, Carboxy oder $C_1$-$C_4$-Alkoxy-carbonyl substituiert ist, und
X für Wasserstoff, Chlor, Methyl oder Trifluormethyl steht.

4. Verfahren zur Herstellung von nitrosubstituierten Benzthiazolonen der allgemeinen Formel (I)

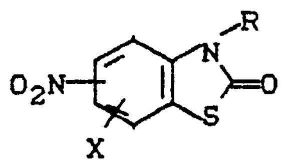

(I)

in welcher
R für Alkyl steht, welches einfach oder zweifach, gleich oder verschieden durch Cyano, Carboxy, Alkylcarbonyl, Alkoxycarbonyl und Alkylaminocarbonyl oder durch jeweils gegebenenfalls ein- bis fünffach, gleich oder verschieden durch Halogen, Alkyl, Halogenalkyl und Alkoxy substituiertes Phenyl oder Phenylcarbonyl substituiert ist, oder für den Rest -CONHR$^1$ steht, worin R$^1$ für Alkyl steht, welches im Alkylteil durch Cyano, Carboxy oder Alkoxycarbonyl substiuiert ist, und
X für Wasserstoff, Halogen, Alkyl oder Halogenalkyl steht, dadurch gekennzeichnet, daß man

(a) nitrosubstituierte Benzthiazolone der allgemeinen Formel (II)

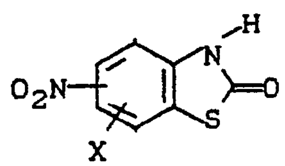

(II)

in welcher
X die oben angegebene Bedeutung hat,
- oder Alkalimetallsalze von Verbindungen der allgemeinen Formel (II) -
mit Halogenverbindungen der allgemeinen Formel (III)
X$^1$-R (III)
in welcher
R die oben angegebene Bedeutung hat und
X$^1$ für Halogen steht,
gegebenenfalls in Gegenwart eines Säureakzeptors und gegebenenfalls in Gegenwart eines Verdünnungsmittels umsetzt, oder daß man

(b) nitrosubstituierte Benzthiazol-3-oxide der allgemeinen Formel (IV)

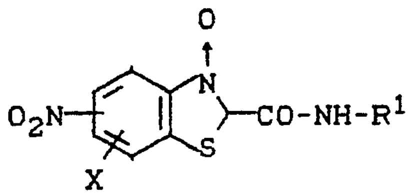

(IV)

in welcher
X und $R^1$ die oben angegebenen Bedeutungen haben,
mit Phosphorylchlorid gegebenenfalls in Gegenwart eines Verdünnungsmittels umsetzt.

5. Fungizide Mittel, gekennzeichnet durch einen Gehalt an mindestens einem nitrosubstituierten Benzthiazolon der Formel (I) gemäß den Ansprüchen 1 bis 4.

6. Verfahren zur Bekämpfung von Pilzen, dadurch gekennzeichnet, daß man nitrosubstituierte Benzthiazolone der Formel (I) gemäß den Ansprüchen 1 bis 4 auf die Pilze und/oder ihren Lebensraum einwirken läßt.

7. Verwendung von nitrosubstituierten Benzthiazolonen der Formel (I) gemäß den Ansprüchen 1 bis 4 zur Bekämpfung von Pilzen.

8. Verfahren zur Herstellung von fungiziden Mitteln, dadurch gekennzeichnet, daß man nitrosubstituierte Benzthiazolone der Formel (I) gemäß den Ansprüchen 1 bis 4 mit Streckmitteln und/oder oberflächenaktiven Substanzen vermischt.

9. Nitrosubstituierte Benzthiazol-3-oxide der Formel (IV)

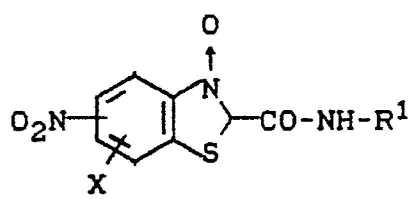

(IV)

in welcher
X für Wasserstoff, Halogen, Alkyl oder Halogenalkyl steht und
$R^1$ für Alkyl steht, welches ein- oder zweifach, gleich oder verschieden durch Cyan, Carboxy und Alkoxycarbonyl substituiert ist.

10. Verfahren zur Herstellung von nitrosubstituierten Benzthiazol-3-oxiden der Formel (IV) gemäß Anspruch 9, dadurch gekennzeichnet, daß man 2-Alkoxycarbonyl-benzthiazol-3-oxide der Formel (V)

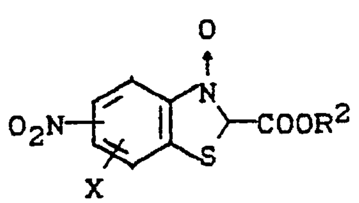

(V)

in welcher
X für Wasserstoff, Halogen, Alkyl oder Halogenalkyl steht und
$R^2$ für Alkyl steht, mit Aminen der Formel (VI)
$H_2N-R^1$ VI)
in welcher
$R^1$ für Alkyl steht, welcher ein- oder zweifach, gleich oder verschieden durch Cyan, Carboxy und Alkoxycarbonyl substituiert ist, gegebenenfalls in Gegenwart eines Verdünnungsmittels umsetzt.

11. 2-Alkoxycarbonyl-benzthiazol-3-oxide der Formel (V)

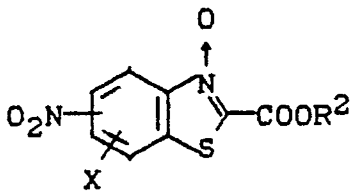

(V)

in welcher
X für Wasserstoff, Halogen, Alkyl oder Halogenalkyl steht und
$R^2$ für Alkyl steht.

12. Verfahren zur Herstellung von 2-Alkoxycarbonylbenzthiazol-3-oxiden der Formel (V) gemäß Anspruch 11, dadurch gekennzeichnet, daß man 2-Chlor-nitrobenzol-Derivate der Formel (VII)

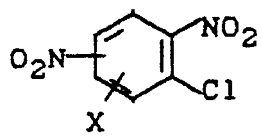

(VII)

in welcher
X für Wasserstoff, Halogen, Alkyl oder Halogenalkyl steht, mit Mercaptoessigsäureestern der Formel (VIII)
$HS\text{-}CH_2\text{-}COOR^2$ (VIII)
in welcher
$R^2$ für Alkyl steht,
in Gegenwart einer Base und in Gegenwart eines Verdünnungsmittels umsetzt.

13. Verwendung der Verbindungen der Formeln (IV) und (V) als Zwischenprodukte.

Europäisches Patentamt

# EUROPÄISCHER RECHERCHENBERICHT

Nummer der Anmeldung

EP 90105653.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.⁵) |
|---|---|---|---|
| A | EP - A1 - 0 218 972 (SUMITOMO CHEMICAL COMPANY, LIMITED) * Ansprüche 1,8-13; Seiten 28-31 (Tabelle 4) * -- | 1,4-13 | C 07 D 277/68<br>A 01 N 43/78 |
| A | EP - A1 - 0 296 416 (NIHON TOKUSHU NOYAKU SEIZO K.K.) * Ansprüche 1,9,10; Seite 14, Verb. (XII); Seite 15, Verb. (XIV) * -- | 1,4-13 | |
| A | CHEMICAL ABSTRACTS, Band 104, Nr. 5, 3. Februar 1986, Columbus, Ohio, USA IDEMITSU KOSAN CO. LTD. "Benzothiazolones as fungicides " Seite 216, Spalte 2, Zusammenfassung Nr. 30 422x & Jpn. Kokai Tokkyo Koho JP 60 105 604 (85 105 604) -- | 1,4-13 | RECHERCHIERTE SACHGEBIETE (Int Cl.⁵) |
| A | CHEMICAL ABSTRACTS, Band 103, Nr. 19, 11. November 1985, Columbus, Ohio, USA IDEMITSU KOSAN CO.LTD. "Benzothiazolinunes" Seite 712, Spalte 2, Zusammenfassung Nr. 160 499t & Jpn. Kokai Tokkyo Koho JP 60 105 671 (85 105 671) ---- | 1,4-13 | C 07 D 277/00 |

Der vorliegende Recherchenbericht wurde für alle Patentansprüche erstellt.

| Recherchenort | Abschlußdatum der Recherche | Prüfer |
|---|---|---|
| WIEN | 15-05-1990 | BRUS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82